(19) Europäisches Patentamt
European Patent Office
Office européen des brevets

(11) Veröffentlichungsnummer: **0 018 482**
**A1**

(12) **EUROPÄISCHE PATENTANMELDUNG**

(21) Anmeldenummer: **80101193.3**

(22) Anmeldetag: **10.03.80**

(51) Int. Cl.$^3$: **C 07 C 43/11**
C 07 C 41/03, C 11 D 1/722
C 11 D 1/825, C 08 G 65/08

(30) Priorität: **04.05.79 DE 2918047**

(43) Veröffentlichungstag der Anmeldung:
**12.11.80 Patentblatt 80/23**

(84) Benannte Vertragsstaaten:
**FR GB IT NL**

(71) Anmelder: **CHEMISCHE WERKE HÜLS AG**
**Postfach 1320**
**D-4370 Marl 1(DE)**

(72) Erfinder: **Kosswig, Kurt, Dr.**
**Hoechster Strasse 10**
**D-4370 Marl(DE)**

(72) Erfinder: **Wienhöfer, Ekkehard, Dr.**
**Griesheimer Strasse 12**
**D-4370 Marl(DE)**

(54) **Biologisch abbaubare und schwachschäumende Tenside, Verfahren zu ihrer Herstellung und ihre Verwendung in Reinigungsmitteln.**

(57) Biologisch abbaubare, schwachschäumende Tenside der Formeln I und/oder II

$$R[O-(\underset{R'}{CH}-\underset{R''}{CHO})_x-(CH_2CH_2O)_y-H]_{1-2} \qquad I$$

$$R\left[O[(\underset{R'}{CH}-\underset{R''}{CHO})_x-(CH_2CH_2O)_y]_z-H\right]_{1-2} \qquad II,$$ wobei

R = 8 bis 22 C-Atome in der Alkylkette enthaltender Alkyl-oder 2 bis 22 C-Atome enthaltender Hydroxialkylrest und R', R'' Wasserstoff oder ein $C_1$-$C_{20}$-Alkylrest ist, wobei R' und R'' nicht gleichzeitig Wasserstoff sind und R' und R'' insgesamt 8 bis 20 Kohlenstoffatome aufweisen, und wobei x in I einen Wert von 0,5 bis 5 und y einen Wert von 5 bis 50 hat und z in II frei wählbar ist mit der Maßgabe, daß die Summe aller x(z) einen Wert von 0,5 bis 5 und die Summe aller y(z) einen Wert von 5 bis 50 hat; sowie Verfahren zur Herstellung dieser Tenside und ihre Verwendung in flüssigen Reinigungsmitteln für maschinelles Spülen.

EP 0 018 482 A1

Biologisch abbaubare und schwachschäumende Tenside, Verfahren zu ihrer Herstellung und ihre Verwendung in Reinigungsmitteln

Schwachschäumende Tenside, die gute Netz- und Reinigungswirkung zeigen, werden in Haushaltsgeschirrspülmaschinen, aber auch für einen entsprechenden Einsatz im gewerblichen Bereich benötigt. So enthalten z. B. feste pulverförmige Reinigungsmittel für Spülmaschinen neben üblichen Bestandteilen wie Pentanatriumtriphosphat, Natriumsilikat, -carbonat und -aluminat, chlorabspaltenden Verbindungen und Riechstoffen oft 1 bis 3 Gewichtsprozent solcher Tenside. In flüssigen Kompositionen für die maschinelle Reinigung sowie in Zusätzen für die Klar- und Glanzspülung können sogar 15 bis 25 Gewichtsprozent enthalten sein.

Tenside, welche die obengenannten Voraussetzungen erfüllen, sind beispielsweise Propylenoxid-Ethylenoxid-Polymerisate, die unter dem Handelsnamen "PLURONICS®" bekannt geworden sind (US-PS 2 674 619). In ihnen kommt den aus dem Propylenoxid stammenden Molekülgerüstteilen der lipophile Charakter zu, während die vom Ethylenoxid gebildeten Segmente als hydrophil anzusehen sind.

Die nach diesem Prinzip aufgebauten Tenside haben allerdings den großen Nachteil, nur sehr schwer biologisch abbaubar zu sein. In einer Zeit, da dem Schutz der Umwelt in allen Bereichen steigende Bedeutung zuerkannt wird,

muß diese Eigenschaft zu starken Beschränkungen in der Anwendbarkeit der genannten Tenside führen. So wird z. B. in der Bundesrepublik Deutschland gemäß dem "Gesetz über die Umweltverträglichkeit von Wasch- und Reinigungsmitteln" vom 20.08.1975 ("Waschmittelgesetz") und der dazu erlassenen "Verordnung über die Abbaubarkeit anionischer und nichtionischer grenzflächenaktiver Stoffe in Wasch- und Reinigungsmitteln" vom 30.01.1977 eine biologische Abbaubarkeit von mindestens 80 % gefordert. Als Kriterium gilt der sogenannte OECD-Confirmatory-Test, durch den der Restgehalt einer Probelösung an nichtionischen, grenzflächenaktiven Stoffen anhand der Wismutaktivität nach Wickbold bestimmt wird. Obwohl die Ethylenoxid-Propylenoxid-Block- und Mischpolymerisate diese Anforderung nicht erfüllen, wurde ihre Verwendung zunächst für drei weitere Jahre genehmigt, in denen nach einem Ersatz gesucht werden soll.

Neben diesen Polymerisaten, die nur Ethylenoxid und Propylenoxid oder gegebenenfalls Diamine enthalten, findet eine weitere Stoffklasse Anwendung als schaumarme Tenside für die obengenannten Gebiete, die als einseitig blockierte Oligomere aus Ethylenoxid-Propylenoxid-Einheiten angesehen werden können (DE-OS 15 93 043). Man gelangt zu diesen Verbindungen durch Addition der Alkylenoxide an geeignete Starter mit aktiven Wasserstoffatomen wie Fettalkohole oder Alkylphenole. Auch hier läßt sich durch Wahl von Block- oder Mischaddition der Charakter des erhaltenen Tensids in Grenzen variieren. Der Vorteil dieser grenzflächenaktiven Stoffe liegt in ihrer gegenüber den "PLURONIC"-Typen verbesserten biologischen Angreifbarkeit. Sie erfüllen die Forderungen des OECD-Confirmatory-Tests; bei einer Prüfung des Grades der völligen Remineralisierung zeigen sie sich jedoch verbesserungswürdig. Will man nämlich vermeiden, daß durch Entstehen von Metaboliten, die ihrerseits einem weiteren Abbau schwer zugänglich oder sogar toxisch sind, eine Gefährdung auftreten kann, so muß der Einsatz von möglichst weitgehend remineralisierbaren Stoffen bevorzugt werden. Remineralisierung bedeutet, daß z. B. eine aus Kohlenstoff, Wasserstoff und Sauerstoff aufgebaute Verbindung, wie sie etwa die meisten nichtionischen Tenside darstellen, in Kohlendioxid und Wasser umgewandelt wird. Die Forderung nach zufriedenstellender Remineralisierung wird dann erfüllt, wenn im biologischen Abbautest, welcher auf den Grad der völligen Remineralisierung abzielt, Werte von 50 % und mehr erreicht werden. Dieser Zustand wird aber von Propylenoxid- und noch ausgeprägter von Butylenoxidaddukten nur in äußerst langsamer Reaktion erreicht, wie Untersuchungen von Fischer (VI. Int. Kongr. grenzfl.akt. Stoffe, Zürich 1972 Band 3, Seite 745 ff, insbesondere Seite 747) zeigen. Die bisher als schaumarme Tenside eingesetzten Abkömmlinge dieser Epoxide stellen also von der Ökologie her keine befriedigende Lösung des Problems dar. Es bestand daher die Aufgabe, schwachschäumende und für den Einsatz in gewerblichen und Haushalts-Spülmaschinen geeignete Tenside zur Verfügung zu stellen, deren Abbaubarkeit, berechnet als Grad der völligen Remineralisierung, zufriedenstellend ist.

Diese Aufgabe wird durch die in den Ansprüchen beschriebene Stoffklasse und das Verfahren zu ihrer Herstellung gelöst. Es muß dabei als besonders überraschend angesehen werden, daß eine wesentliche Vergrößerung des Alkylrestes im addierten 1.2-Epoxialkan zu guter bis sehr guter Remineralisierbarkeit der neuen Tenside führt, obwohl aus der o. a. Literaturstelle Fischer bekannt war, daß Butylenoxidaddukte eine geringere Abbaubarkeit zeigen als Propylenoxidaddukte. Bei der Entwicklung des Gegenstandes der vorliegenden Erfindung bedurfte es daher zur Überwindung des bestehenden Vorurteils eines ansehnlichen erfinderischen Aufwandes.

Für das erfindungsgemäße Verfahren geeignete Verbindungen $R(OH)_{1-2}$ sind z. B. geradkettige und verzweigte, gegebenenfalls substituierte primäre und sekundäre Alkohole mit 8

bis 22 C-Atomen wie 1-Octanol, 2-Octanol, 1-Decanol, 1-Undecanol, 2-Undecanol, 1-Dodecanol, 1-Tridecanol, 2-Tridecanol, 1-Tetradecanol, 1-Hexadecanol, 1-Octadecanol, 1-Eicosanol, 1-Docosanol, 2-Ethylhexanol; Gemische von Alkoholen nativen Ursprungs; Alkohole aus der Aufbaureaktion nach Ziegler und anschließender Oxidation oder deren Gemische sowie die Produkte der Hydroformylierung oder Hydrocarboxymethylierung von end- und innenständigen Olefinen und nachfolgender Hydrierung; 1,2-, 1,3- und $\alpha,\omega$-Alkandiole mit 2 bis 22 C-Atomen wie Ethylenglykol, Propylenglykol, 1,3-Propandiol, 1,3-Butandiol, 1,4-Butandiol, 1,2-Hexandiol, 1,2-Octandiol, 1,2 Decandiol, 1,2-Dodecandiol, 1,2-Tetradecandiol, 1,2-Hexadecandiol, 1,2-Octadecandiol, 1,2 Eicosandiol, 1,2-Docosandiol.

Als Katalysatoren zur Anlagerung der Epoxide R'-CH-CH-R"
      \ /
       O
und des Ethylenoxids werden bevorzugt basische Verbindungen wie Kaliumhydroxid, Natriumhydroxid oder entsprechende Alkoholate mit 1 bis 4 C-Atomen wie Natriummethylat, Natriumethylat oder Kalium-tert.-butanolat. Es lassen sich aber auch saure Katalysatoren wie Bortrifluorid oder komplexe Oxonium- oder Carbeniumsalze, beispielsweise Triethyloxonium-tetrafluoroborat einsetzen.

Als erfindungsgemäß geeignete Epoxidkomponenten der Formel R'-CH-CH-R" sind 1,2-Epoxide mit 10 bis 22 Kohlen-
      \ /
       O
stoffatomen, mittelständige Epoxide der gleichen Kohlenstoffzahl sowie Gemische daraus und die Epoxidationsprodukte 2-alkylsubstituierter $\alpha$-Olefine, wie sie bei der aluminiumorganisch katalysierten Dimerisation kurzkettiger $\alpha$-Olefine entstehen, anzusehen. Besonders geeignet sind Epoxide mit 14 bis 22 Kohlenstoffatomen.

Es lassen sich somit z. B. einsetzen 1,2-Epoxidecan, 1,2-Epoxidodecan, 1,2-Epoxitetradecan, 1,2-Epoxihexadecan, 1,2-Epoxi-dodecan/-tetradecan/-hexadecan als Gemisch, 1,2-Epoxioctadecan, 1,2-Epoxieicosan, 5,6-Epoxidodecan, 7,8-Epoxihexadecan, 7,8-Epoxioctadecan, 1,2-Epoxi-2-butyloctan, 1,2-Epoxi-2-hexyldecan, 1,2-Epoxi-2-ethyldodecan sowie das Produkt der Umsetzung eines Gemisches von mittelständigen Olefinen mit 11 bis 15 Kohlenstoffatomen mit einer geeigneten Percarbonsäure.

Die Mengenverhältnisse von Hydroxikohlenwasserstoff, Epoxid und Ethylenoxid untereinander sollten so beschaffen sein, daß 20 bis 80, bevorzugt 30 bis 70 und besonders bevorzugt 40 bis 60 Gewichtsprozent des Moleküls lipophil, der Rest dagegen hydrophil ist. Lipophil sind die aus dem als Starter eingesetzten Hydroxikohlenwasserstoff und aus dem Epoxid $R'-\underbrace{CH-CH}_{O}-R''$ stammenden Teile des Moleküls; die aus dem Ethylenoxid stammenden Segmente dagegen sind hydrophil. Ein aus 1-Dodecanol, 3 Mol 1,2-Epoxitetradecan und 30 Mol Ethylenoxid zusammengesetztes Tensid hat somit ein entsprechendes Verhältnis von 38 % zu 62 %, liegt also im bevorzugten Bereich.

Bei Verwendung der bevorzugten primären Alkohole aus dem Fettalkylbereich ($C_8$-$C_{22}$) als Starter und der aus dem C-Zahl-Bereich ($C_{10}$-$C_{22}$) stammenden 1,2-Epoxialkane sowie bei Beachtung des bevorzugten Verhältnisses zwischen lipophilem und hydrophilem Anteil im gewünschten Endprodukt liegen die Molekulargewichte der so erzeugten Tenside zwischen 800 und 2 500. Werden für besondere Zwecke spezielle Alkohole oder Epoxide benötigt, können die Molekulargewichte natürlich deutlich außerhalb des genannten Bereichs liegen.

Die Addition der Alkylenoxidkomponenten an den Starter $R(OH)_{1-2}$ kann so gelenkt werden, daß entweder Block- oder Mischaddukte entstehen. Während die zum Blockaddukt führende primäre Umsetzung des Starters mit einem 1,2-Epoxialkan mit nachfolgender Ethylenoxidaddition in der ersten Stufe Temperaturen von 210 bis 230 °C, in der zweiten dagegen nur solche von 150 bis 170 °C benötigt, um in einer befriedigenden Zeit die gewünschte Reaktion durchzuführen, genügt für die das Mischaddukt ergebende gleichzeitige Umsetzung des Starters mit Epoxid und Ethylenoxid eine Temperatur von 180 bis 200 °C, da die Reaktion des Epoxids $R'-\underbrace{CH-CH}_{O}-R''$ von der Anwesenheit des sehr reaktiven Ethylenoxids profitiert. Die Umsetzung kann bei Normaldruck ablaufen, jedoch kann vorteilhaft ein Überdruck von 0,5 bis 3 at angewendet werden.

Die Blockaddukte erweisen sich als etwas besser wasserlöslich; die Fähigkeit, die Oberflächenspannung von Wasser zu senken, ist aber für beide Stoffgruppen gleich ausgeprägt und erreicht für 2 %ige Lösungen in vollentsalztem Wasser Werte von 30 bis 35 mN/m, wie sie auch bei schon bekannten Tensiden üblich sind.

Ihren besonderen Vorzug beweisen die neuen Tenside im biologischen Abbautest. Während sie einerseits wie alle oberflächenaktiven Stoffe, die eine n-Alkyl-Kette mit einer C-Zahl etwa des Fettalkylbereichs besitzen, den OECD-Confirmatory-Test mit einer Rate von $>90$ % Schwund an Wismutaktiver Substanz sehr gut bestehen, weisen sie auch einen überraschend hohen Grad an Remineralisierbarkeit auf. Nach der "dissolved organic carbon" (DOC)-Methode im Ablauf einer Modellkläranlage im sogenannten "coupled units"-Test gemessen, finden sich Werte zwischen 50 und 80 %, wovon der obere Bereich von den Blockaddukten, der untere von den Mischaddukten gebildet wird. Im deutlichen Gegensatz dazu stehen die Befunde, die in der Literatur (W. K. Fischer, s. o.) über die Remineralisierbarkeit von Addukten des Propylen- und Butylenoxids an Fettalkohole und -alkoholoxethylate zugänglich sind. Im Vergleich mit den nach dem Stand der Technik bevorzugt als schaumarme Tenside eingesetzten Propylenoxidderivaten müssen die erfindungsgemäßen Produkte also von der Ökologie her als deutlich überlegen angesehen werden.

Die folgenden Beispiele erläutern die Methoden zur Darstellung und die Wirkung der neuen Tenside.

Beispiel 1

22,6 g (0,121 Mol) 1-Dodecanol werden mit 1,0 g pulverisiertem Natriumhydroxid versetzt und auf 160 °C erwärmt. Nachdem eine klare Lösung entstanden ist, werden 77,4 g (0,365 Mol) 1,2-Tetradecanepoxid zugetropft. Die Lösung wird dann 12 Stunden bei dieser Temperatur gerührt, bis der Epoxidgehalt unter 5 % gesunken ist. Innerhalb einer Stunde werden anschließend 106,5 g (2,420 Mol) Ethylenoxid bei 170 °C eingegast, die in exothermer Reaktion aufgenommen werden. Es resultiert ein pastöses gelbliches Produkt, das gegebenenfalls mit 1 bis 3 % Wasserstoffperoxid unter Zusatz einer Mineralsäure gebleicht werden kann. Ein Gehalt an Restalkohol ist nicht festzustellen; der Polydiolanteil im Produkt beträgt 4,6 %.

Im DOC-Test zeigt sich, daß das Produkt zu 79,4 ± 4,8 % remineralisiert wird, während im OECD-Confirmatory-Test eine Abbaubarkeit von > 90 % gefunden wird.

Beispiel 2

67,8 g (0,366 Mol) 1-Dodecanol werden in Gegenwart von 1,4 g pulverisiertem Natriumhydroxid mit 232,2 g (1,094 Mol) 1,2-Tetradecanepoxid bei 200 °C umgesetzt. Nach 2,5 Stunden ist der Epoxidgehalt der Lösung auf 2,6 % gesunken. Die weitere Addition von 372,0 g (8,454 Mol) Ethylenoxid bei 180 bis 200 °C ergibt ein Produkt mit 7,2 % Polydiolgehalt.

Beispiel 3

72,5 g (0,25 Mol) eines Fettalkoholgemisches mit der mittleren Molmasse 290 werden mit 0,9 g Natriumhydroxid bei 220 °C gerührt, bis eine klare Lösung vorliegt. Bei 200 °C

wird mit der Zugabe von Ethylenoxid als Gas begonnen; nach Anspringen der Oxethylierung werden innerhalb von 2 Stunden 78,1 g (0,50 Mol) 1,2-Epoxidecan und 330,0 g (7,50 Mol) Ethylenoxid gleichzeitig zugegeben, die letzten 5 ml des Epoxids allerdings erst nach beendeter Ethylenoxidzufuhr. Dann wird bei 210 °C noch 1 Stunde nachgerührt. Es werden ein Restalkoholwert von <0,1 % und ein Polydiolanteil von 1,0 % gefunden. Im DOC-Abbau-Test zeigt sich, daß das Produkt zu 56,2 ± 6,1 % remineralisiert wird, während im OECD-Confirmatory-Test eine Abbaubarkeit von >90 % gefunden wird.

Beispiel 4

Die nach den Beispielen 1 bis 3 hergestellten Produkte werden in die nachfolgend genannten Formulierungen von für Geschirrspülautomaten geeigneten Spülmitteln gebracht und im Normalspülgang eines herkömmlichen Fabrikats mit zwei im Handel befindlichen Spülmitteln verglichen (eingesetzte Menge an Spülmittel: 15 g; Arbeitstemperatur 60 °C).

Formulierung I:

| | |
|---|---|
| Tensid nach Beispiel 1 | 3 Gew.-% |
| Pentanatriumtriphosphat | 50 Gew.-% |
| Natriummetasilikat | 20 Gew.-% |
| Natriumperborat | 25 Gew.-% |
| Natriumaluminat | 2 Gew.-% |

Formulierung II:

| | |
|---|---|
| Tensid nach Beispiel 2 | 3 Gew.-% |
| Pentanatriumtriphosphat | 70 Gew.-% |
| Natriummetasilikat | 23 Gew.-% |
| Natriumaluminat | 2 Gew.-% |
| Natriumdichlorisocyanurat | 2 Gew.-% |

Formulierung III:

| | |
|---|---|
| Tensid nach Beispiel 3 | 3 Gew.-% |
| Natriumcarbonat calc. | 20 Gew.-% |

| | |
|---|---|
| Pentanatriumtriphosphat | 50 Gew.-% |
| Natriummetasilikat | 23 Gew.-% |
| Natriumaluminat | 2 Gew.-% |
| Natriumdichlorisocyanurat | 2 Gew.-% |

Tabelle 1 zeigt die an Gegenständen des täglichen Gebrauchs eines 4-Personen-Haushalts erzielten Reinigungswerte als Gegenüberstellung zwischen zufriedenstellend gereinigten und insgesamt behandelten Teilen. Die Schaumhöhe in der Maschine wurde jeweils 20 min nach Beginn des Waschgangs festgestellt und betrug einheitlich 4 bis 5 cm über der Spülflotte.

Tabelle 1

Reinigungswirkung von Test- und Vergleichsspülmitteln

| | Eßteller | Tassen | Untertassen | Messer |
|---|---|---|---|---|
| Formulierung I | 18/18 | 14/16 | 16/16 | 11/13 |
| Formulierung II | 18/19 | 16/16 | 14/14 | 10/12 |
| Formulierung III | 18/18 | 15/16 | 15/16 | 11/12 |
| Käufe, Spülmittel A | 17/17 | 14/14 | 14/14 | 12/14 |
| Käufe, Spülmittel B | 17/18 | 13/15 | 15/15 | 12/12 |

Fortsetzung Tabelle 1

| | Gabel | Löffel | Wassergläser |
|---|---|---|---|
| Formulierung I | 12/14 | 12/12 | 7/7 |
| Formulierung II | 14/15 | 8/8 | 8/8 |
| Formulierung III | 12/12 | 13/15 | 6/7 |
| Käufe, Spülmittel A | 14/14 | 10/12 | 7/7 |
| Käufe, Spülmittel B | 14/15 | 10/12 | 7/8 |

Beispiel 5

In Tabelle 2 sind die Werte der Oberflächenspannung von 2 %igen Lösungen erfindungsgemäßer Tenside gegenüber Luft

wiedergegeben. Bei allen Produkten handelt es sich um Addukte aus 1-Dodecanol, 1,2-Epoxitetradecan und Ethylenoxid, die nach dem Verfahren aus Beispiel 1 erhalten wurden. Zum Vergleich ist der Wert eines handelsüblichen Nonylphenolderivates mit Propylen- und Ethylenglykolethergruppen mit angegeben.

Tabelle 2

Oberflächenspannung einer 2 %igen Tensidlösung in VE-Wasser

| Nr. | Anteil | | Oberflächenspannung |
|---|---|---|---|
| | Epoxid | EO | (mN/m) |
| 1 | 2 | 19 | 35,5 |
| 2 | 2 | 22 | 36,5 |
| 3 | 2 | 25 | 39,5 |
| 4 | 3 | 18 | 34 |
| 5 | 3 | 25 | 38,5 |
| 6 | 3 | 29 | 37 |
| Vergleich | 10 PO | 9 | 34 |

Beispiel 6

In Tabelle 3 sind die Trübungspunkte erfindungsgemäßer Tenside sowie die Schaumhöhen 2 %iger Lösungen in VE-Wasser bei 20 °C nach 0, 5 und 10 Min. angegeben. Dazu wurde zunächst in einem Schliffstopfen-verschlossenen Meßzylinder von 50 ml Inhalt die Probelösung zehnmal geschüttelt und dann die Höhe des so erzeugten Schaumes in Abhängigkeit von der Zeit gemessen. Zum Vergleich sind die Meßwerte eines schaumarmen Nonylphenol-EO-PO-Adduktes mit aufgeführt. Die Adduktypen sind mit I (Blockaddukt; Beispiel 1) und II (Mischaddukt; Beispiel 3) gekennzeichnet. Tabelle 3 siehe Seite 11.

Tabelle 3

Trübungspunkte und Schaumhöhen erfindungsgemäßer Tenside

| Nr. | Alkoholkomponente | α-Epoxid/Anzahl | | EO | Addukt-typ | Trüb.-Pkt.*) (°C) | Schaumhöhen (cm) nach | | |
|---|---|---|---|---|---|---|---|---|---|
| | | | | | | | 0 min | 5 min | 10 min |
| 1 | 1-Dodecanol | $C_{14}H_{28}O$/ | 2 | 16 | I | 88 | 6,1 | 4,0 | 3,8 |
| 2 | 1-Dodecanol | $C_{14}H_{28}O$/ | 2 | 26 | II | 61 | 7,5 | 3,6 | 2,9 |
| 3 | Alkoholmischung C 10/12 | $C_{14}H_{28}O$/ | 2 | 23 | II | 60 | 7,5 | 5,0 | 4,0 |
| 4 | Alkoholmischung C 10/12 | $C_{14}H_{28}O$/ | 3 | 30 | II | 58 | 7,8 | 4,8 | 4,1 |
| 5 | Alkoholmischung C 10/12 | $C_{10}H_{20}O$/ | 2 | 23 | II | 61 | 7,8 | 4,5 | 2,2 |
| 6 | Alkoholmischung C 10/12 | $C_{10}H_{20}O$/ | 3 | 28 | II | 53 | 9,5 | 6,5 | 3,0 |
| 7 | Alkoholmischung C 16/20 | $C_{10}H_{20}O$/ | 2 | 20 | I | trübe | 6,5 | 2,7 | 2,4 |
| 8 | Alkoholmischung C 16/20 | $C_{10}H_{20}O$/ | 2 | 30 | II | 63 | 7,0 | 2,0 | 1,3 |
| 9 | 1,2-Tetradecandiol | $C_{14}H_{28}O$/ | 3 | 34 | II | 53 | 10,0 | 5,0 | 3,2 |
| 10 | 1,4-Butandiol | $C_{14}H_{28}O$/ | 4 | 24 | II | 40 | 6,5 | 5,3 | 4,7 |
| Vergleich | Nonylphenol | $C_3H_6O$/ | 10 | 9 | I | - | 7,0 | 1,2 | 0,6 |

*) aus 10 %iger Lösung des Tensids in 25 %iges Butyldiglykol/VE-Wasser

0018482

Patentansprüche:

1. Biologisch abbaubare und schwachschäumende Tenside der Formeln I und/oder II

$$R[O-(\underset{R'}{CH}-\underset{R''}{CH}O)_x-(CH_2CH_2O)_y-H]_{1-2} \qquad \text{I}$$

$$R\left[O[(\underset{R'}{CH}-\underset{R''}{CH}O)_x-(CH_2CH_2O)_y]_z-H\right]_{1-2} \qquad \text{II}$$

dadurch gekennzeichnet,
daß R einen 8 bis 22 C-Atome in der Alkylkette enthaltenden Alkyl- oder einen 2 bis 22 C-Atome enthaltenden Hydroxialkylrest und R', R" Wasserstoff oder einen $C_1$-$C_{20}$-Alkylrest bedeuten, wobei R' und R" nicht gleichzeitig Wasserstoff sind und R' und R" insgesamt 8 bis 20 Kohlenstoffatome aufweisen, daß in I x einen Wert von 0,5 bis 5 und y einen Wert von 5 bis 50 hat und daß in II z frei wählbar ist mit der Maßgabe, daß die Summe aller x(z) einen Wert von 0,5 bis 5 und die Summe aller y(z) einen Wert von 5 bis 50 hat.

2. Biologisch abbaubare und schwachschäumende Tenside nach Anspruch 1,
dadurch gekennzeichnet,
daß in I x einen Wert von 2 bis 3 und y einen Wert von 20 bis 30 hat.

3. Biologisch abbaubare und schwachschäumende Tenside nach Anspruch 1,
dadurch gekennzeichnet,
daß in II die Summe aller x(z) einen Wert von 2 bis 3 und die Summe aller y(z) einen Wert von 20 bis 30 hat.

4. Verfahren zur Herstellung von Tensiden gemäß Anspruch 1,
dadurch gekennzeichnet,

daß man eine Verbindung $R(OH)_{1-2}$ mit aktivem Wasserstoff vorlegt, einen üblichen sauren oder basischen Oxalkylierungskatalysator zufügt und bei einer geeigneten Temperatur mit einem Epoxid $R'-\underbrace{CH-CH}_{O}-R''$ und Ethylenoxid umsetzt.

5. Verfahren gemäß Anspruch 2,
dadurch gekennzeichnet,
daß man zur Herstellung von I die vorgelegte Lösung von Alkohol und Katalysator zunächst nur mit dem Epoxid $R'-\underbrace{CH-CH}_{O}-R''$ und anschließend mit Ethylenoxid umsetzt.

6. Verfahren gemäß Anspruch 2,
dadurch gekennzeichnet,
daß man zur Herstellung von II die vorgelegte Lösung von Alkohol und Katalysator gleichzeitig mit dem Epoxid $R'-\underbrace{CH-CH}_{O}-R''$ und Ethylenoxid umsetzt.

7. Verfahren nach den Ansprüchen 2 bis 4,
dadurch gekennzeichnet,
daß als Alkoholkomponente Fettalkohole mit 8 bis 22 C-Atomen oder Gemische davon oder 1,2-Alkandiole der gleichen C-Kettenlänge eingesetzt werden.

8. Verfahren nach den Ansprüchen 2 bis 5,
dadurch gekennzeichnet,
daß als Epoxidkomponente $R'-\underbrace{CH-CH}_{O}-R''$ 1,2-Alkanepoxide mit 12 bis 16 C-Atomen oder Gemische davon verwendet werden.

9. Verfahren nach den Ansprüchen 2 bis 6,
dadurch gekennzeichnet,
daß die Umsetzung bei 150 bis 230 °C und unter Normaldruck oder Überdruck durchgeführt wird.

10. Verwendung der Tenside nach Anspruch 1 in festen und flüssigen Reinigungsmitteln für maschinelles Spülen in Haushalt und Industrie.

0018482

Europäisches Patentamt

# EUROPÄISCHER RECHERCHENBERICHT

Nummer der Anmeldung
EP 80101193.3

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | AT - B - 336 555 (HENKEL & CIE)<br>+ Ansprüche; Seite 3, Zeilen 38-41 +<br>-- | 1,4,5, 7-10 | C 07 C 43/11<br>C 07 C 41/03<br>C 11 D 1/722<br>C 11 D 1/825<br>C 08 G 65/08 |
| | DE - A1 - 2 432 757 (HENKEL & CIE)<br>+ Seiten 1-4; Beispiele; Ansprüche +<br>-- | 1,4, 8-10 | |
| A | DE - A - 2 318 950 (HENKEL & CIE)<br>+ Seiten 1,2 +<br>-- | 1,2 | RECHERCHIERTE SACHGEBIETE (Int. Cl. 3) |
| | DE - A1 - 2 448 388 (OLIN CORP.)<br>+ Ansprüche +<br>-- | 1,4,5, 7,9 | C 07 C 43/00<br>C 07 C 41/00<br>C 11 D 1/00<br>C 08 G 65/00 |
| | FR - A - 1 063 244 (WYANDOTTE CHEMICALS CORPORATION)<br>+ Ansprüche, Seiten 8,9 +<br>---- | 1,4,5 | |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| WIEN | 09-07-1980 | KÖRBER |